# EUROPEAN PATENT SPECIFICATION

(11) **EP 1 000 088 B1**
(45) Date of publication and mention of the grant of the patent: **27.12.2006**
(21) Application number: 98933610.2
(22) Date of filing: 12.06.1998
(51) Int. Cl.: C07K 16/06, C07K 16/12

(54) **PROCESS FOR THE SEPARATION OF SPECIFIC IMMUNOGLOBULINS FROM STANDARD PLASMA**
VERFAHREN ZUR TRENNUNG SPEZIFISCHER IMMUNOGLOBULINEN AUS STANDARDPLASMA
PROCEDE DE SEPARATION D'IMMUNOGLOBULINES SPECIFIQUES DU PLASMA ORDINAIRE

(30) Priority: 12.06.1997 IT FI970140
(43) Date of publication of application: 17.05.2000
(73) Proprietor: MINISTERO DELL' UNIVERSITA' E DELLA RICERCA SCIENTIFICA E TECNOLOGICA (M.U.R.S.T.), 00144 Roma (IT)
(72) Inventor: ARRIGHI, Silvana, I-53010 Casciano di Murlo (IT); NERI, Paolo, I-53100 Siena (IT)
(74) Representative: Gervasi, Gemma
(86) International application number: PCT/EP1998/003559
(87) International publication number: WO 1998/056822

(56) References cited:
- EP-A- 0 764 658
- US-A- 4 264 449
- WILLIAMS C.A. AND CHASE M.W.: "Methods in immunology and immunochemistry " 1967 , ACADEMIC PRESS , NEW YORK XP002081191 see page 367 - page 372
- MOSBACH K.: "Methods in enzymology vol.135 -immobilized enzymes and cells (part b)-" 1987 , HARCOURT BRACE JOVANOVICH , ORLANDO ETC. XP002081226 see page 160 - page 162

## Description

### Field of Invention

The present invention relates to a process for the production of anti-whooping cough and anti-diphtheria immunoglobulins starting from standard plasma, in which a solution of Cohn's fraction II (obtained from standard plasma) is subjected to affinity chromatography under particular conditions.

### State of the Art

It is known that standard immunoglobulins are normally obtained from standard plasma solutions (i.e. plasma obtained from healthy non-immunized donors) through a fractionating process at low temperature with alcohol (e.g. according to the Cohn's method) of the Cohn's fraction II (immunoglobulin enriched plasma fraction); starting from this fraction, using classic or chromatographic purification techniques, the desired immunoglobulin concentrates are obtained.

Furthermore it is known that, when specific immunoglobulins are desired, in order to obtain their separation according to known techniques, it is necessary to use hyperimmune plasma (i.e. plasma supplied by immunized donors) enriched with the desired immunoglobulin.

Infact, it is impossible to prepare specific immunoglobulins from standard plasma, since the specific immunoglobulin concentration in such a starting product is so low that it is practically impossible to prepare concentrates of suitable titre.

Hyperimmune plasma use results in definitely higher costs compared to standard plasma; moreover, donor immunization is not permitted in certain countries, therefore necessitating the importation of hyperimmune plasma from abroad with a further increasing of costs and practical procedures.

EP- 764 658 describes a process for the separation of various immunoglobulines starting from standard plasma and applying affinity chromatography.

It should be considered that the isolation of specific immunoglobulin from standard plasma using affinity chromatography requires the overcoming of numerous problems, in particular:
a) definition of the quantities and the conditions for the attachment of the antigen to the resin;
b) definition of the optimum attachment conditions of the desired immunoglobulin to the resin-antigen complex;
c) definition of the optimum conditions for the attainment of the quantitative detachment of the desired immunoglobulin during the elution, for the collection of the same immunoglobulin.

### Detailed description of the invention

The present invention makes it possible the isolation of specific immunoglobulins starting from standard plasma, by adopting a method in which a solution of Cohn's fraction II, obtained from standard plasma, is subjected to affinity chromatography under opportune conditions.

As mentioned above, the starting product for the process, according to the invention, is the Cohn's fraction II (obtained according to known techniques). Affinity chromatography is performed on chromatographic columns in which the activated matrix (resin) has been derived with an antigen, capable of recognizing and specifically binding to the desired immunoglobulin.

According to the invention, for example, chemically stable active acrylic resins, capable of forming a link of sufficient stability with the immunoglobulins to be isolated, can be used.

For example, resins are utilized in which the reactive function for the antigen attachment is represented by an oxyranic group, since it reacts electroneutrally with the ligand with no byproduct release; an example of this resin type, useful for the present process is Eupergit C (manufactured by Rhom Pharma) or agarose resins derivatized with a hydrophilic spacer, terminally functionalized, as N-hydroxysuccinimid ester, which allows, in bland conditions, rapid and efficient coupling with antigens containing an aminic group, therefore allowing the formation of very stable covalent links with respect to: denaturants, detergents, heat, solvents, acids and bases (pH range 2-11). These resins also possess excellent mechanical properties that allow elevated flows, and are therefore especially useful for large scale purification processes. One example of this resin type is Affi-Prep10^{®} (manufactured by Bio-Rad).

In particular, the oxyranic group resins have proved preferable in the isolation of diphtheria immunoglobulins; whereas agarose resins are preferred for the isolation of the whooping cough immunoglobulins.

However, it is also obvious that other known similar resins can be used according to the invention. The above mentioned resins are thus derived with a specific antigen for the desired immunoglobulin; such antigens are known and the resin derivation is performed according to known techniques.

Of particular importance is the maximum quantity of antigen that can be bound to the resin per unit volume so as to have maximum immunoglobulin yield, therefore such a quantity does not necessarily correspond to the maximum quantity of antigen which can be theoretically bound to the resin per unit volume.

For whooping cough, the antigen/resin ratio (expressed as mg of antigen per ml of resin) is between 0.8-3 (ideally 1.5); for diphtheria 3-5.5 (ideally 4.8). The resin is packed in a chromatography column; the column height/diameter ratio should ideally be between 1:1 and 3:1; the column is then repeatedly washed with a phosphate buffer (pH 7.4). It is then loaded at a given rate, with the solution obtained by dissolving the Cohn's fraction II, in order to perform the chromatographic separation.

The contact time between derived resin and solution should be approximately 14-18 hours.

The column is then washed with a phosphate buffer solution of almost neutral pH (usually 7.0-7.6) to eliminate all the unbound proteins and then elution follows with a definitely acidic glycin buffer (pH usually 2.0-3.0).

The phosphate buffer is usually 0.1 M and comprises: 0.0031M monobasic potassium phosphate and 0.0069M bibasic potassium phosphate (pH 7.2); the glycin buffer consists of: 0.3 M glycin and HCl q.s. (pH of 2.5).

Operating in this way, it is possible to recuperate the immunoglobulins previously fixed to the substrate in a practically quantitative manner.

Normally 12-16 beds are used for washing (preferably 14).

The elution is conducted with a flow rate of between 3.5 and 7.5 cm/h.

The collected eluate is then neutralized immediately with a phosphate buffer (pH 9) and 40% maltose. Finally the product is filtered against a suitable buffer solution, concentrated and lyophilized according to known techniques. Some examples are reproduced below to provide some indication of the process method according to the invention.

### Example 1

### Isolation of anti-whooping cough immunoglobulins

### Preparation of the Cohn's fraction II

A set of frozen standard plasma bags, equivalent to 2,000 litres are thawed at a temperature of 2-3°C and centrifugated.

The supernatant plasma is adjusted to a pH of 5.8-5.9 with pH 4 acetate buffer (consisting of: 21.7 g/l sodium acetate tryhydrate and 48ml/l acetic acid) and added with 96% ethyl alcohol at -18°C, so as to obtain a final concentration of 19% maintaining the plasma temperature at -5 to -7°C. The whole solution is left under stirring for 6 hours at -7°C and centrifugated. The precipitate (approx. 60 g/l) named Cohn's fraction I + II + III is collected.

About 60kg of paste comprising the above said fraction I + II + III are suspended in water at a temperature of 4°C in a ratio of 1 kg of precipate for every 51t of water. The solution's pH is adjusted to 4.8 with 1 M phosphate buffer (pH 4).

After about 2 hours contact time at a temperature of 2°C-4°C, the solution is further diluted with water at 4°C until a concentration of 15 litres/kg of paste is obtained.

The solution pH is adjusted, under stirring, to 5.05-5.1 with phosphate buffer and topped up to a concentration of 17% with 96% ethylic alcohol.

After contact whilst under stirring, for at least 6 hours, at a temperature of -1 °C the solution is subjected to centrifugation; the precipitate consists of the Cohn's fractions I + III.

The supernatant is subjected to deep layer filtration and NaCl (3g/l) is added, pH adjusted to 7.25 with NaOH (0.3 N) and 96% ethylic alcohol is added up to a concentration of 15%. The suspension is left under stirring for at least 6 hours at 7°C and centrifugated. The collected precipitate (approx. 15g/l of plasma) is the Cohn's fraction II enriched with immunoglobulins (the yield calculated on the initial plasma is approx. 5kg of immunoglobulins per 1,000 I of plasma).

### Preparation of Standard Batches of Cohn's fraction II

4kg of the fraction II obtained as described above, are solubilized at a ratio 1:6 in bibasic sodium phosphate buffer (0.1 M, pH 6.8) and filtered for 0.45µm.

This clear solution is diafiltered, at constant volume, with ultrafiltration system using membranes K 100 (100,000 n.m.w) against three volumes of bibasic sodium phosphate buffer (0.1 M, pH 6.8).

The final solution, adjusted to a concentration of 50g/l is filtered under sterile conditions and then divided into 20 aliquots of 1,200ml each.

### Purification of specific anti-whooping cough immunoglobulins using affinity chromatography

### a) Derivatization of the resin

1,100 ml of Affi-Prep 10^{®}, prepared in accordance with the manufacturer's instructions, are added to 370 ml of whooping cough anatoxin at a proteic concentration of 2.7 mg/ml. The mixture is left under slow stirring for 3 hours at room temperature, and then left to rest overnight at 4°C.

40 ml of 1 M glycin buffer (pH8) are added and the solution is left under slow stirring for 1 hour at room temperature. The mixture is filtered and washed with 5 volumes of 0.01 M phosphate buffer containing 0.15 M NaCl (pH 7.4). Protein dosage, performed on the resins washing solutions, reveals that 1 mg of protein has been bound for 1 ml of resin.

The anatoxin/resin binding is stabilized by treatment with glutaraldehyde.

A buffer containing 0.1 M phosphate, 0.5 M NaCl, 0.05% glutaraldehyde (pH 7.6) is added to the resin derivatized with anatoxin. It is left under slow stirring for 2 hours at room temperature and then 0.02 M glycin buffer is added to the final solution. The resin is then filter washed with 0.1 M phosphate buffer containing 0.02 M glycin.

### b) Affinity Chromatography

The derivatized and stabilized resin (as described above) is packed into a column 11cm x 11 cm (d/h). Before every chromatography the column is treated with:
- 15 beds of buffer T1 (for the initial washing)
- 7 beds of buffer T2 for the blank elution
- 15 beds of buffer T1 for the conditioning

Buffer T1 consists of:
0.01 M Phosphate buffer
0.15 M NaCl
pH 7.4

Buffer T2 (glycin buffer) consists of :
0.3M glycin
HCl q.s. (pH 2.5)

An aliquot of solution containing immunoglobulin (prepared as previously described) is loaded at a rate of 0.8 cm/h, for a contact time of 16 hours. It is washed with 15 beds of T1 buffer. Elution is performed at a flow rate of 6.7 cm/h with buffer T2, collecting the corresponding solutions at the registered elution peak. The eluate is collected and the pH is immediately neutralized with 1 M K2HP04 (pH 9) and 40% maltose obtaining a solution of pH > 6 and 10% maltose.

The eluate solution, diafiltered against 5 volumes of sterile and apyrogenic distilled water with ultrafiltration system with a membrane cut-off 100,000 n.m.w., is added with 22 g/l glycin and 3g/l NaCl maintaining the pH at 6.8+-0.1.

The chromatographic process carried out in this manner allows to recover 6,000-6,500 UELISA/I of plasma (approx. 40% of the load).

### Example 2

### Isolation of anti-diphtheria immunoglobulins

The Cohn's fraction II which constitutes the starting product is prepared as described in Example 1.

### Purification of specific anti-diphtheria specific immunoglobulins using affinity chromatography

### a) Derivation of the resin

2g of Eupergit C are directly expanded in 8 ml of diphtheric anatoxin solution (CRM 197) at a proteic concentration of 5,4 mg/ml and with a titrate (flocculants/ml) of approx. 1,874 Lf/ml. The derivatized resin is packed in a 16 x 45 mm (d/h) column. A total volume of 9 ml of resin in the column with 4.8 mg of total proteins/ml of resin, equal to 1,667 If/ml is obtained.

### b) Affinity Chromatography

The column is treated with:
- 7 beds of buffer T3 for the first washing
- 7 beds of buffer T2 for the blank elution
- 7 beds of buffer T3 for the conditioning

The buffers T2 and T3 are as described previously.

Portions of sample aliquots (prepared as previously) are loaded, 28.5 mg of total proteins/ml of resin, at a rate of 0.117 cm/h, for a contact time of 16 hours. It is washed with 14 beds of buffer T1. Elution is performed at a flow rate of 7.5 cm/h with buffer T2, collecting the corresponding solutions at the registered elution peak.

The eluate is collected in fractions of 6 ml each and immediately neutralized with 2 ml of 1M K2HP04 (pH 9) and 40% maltose, obtaining a solution of pH > 6, and 10% maltose. The yield obtained is 30% of the load.

Obviously, the isolation processes of the various immunoglobulins separately described above can be carried out in sequence, transferring the eluate after the isolation of an immunoglobulin in a column and at the conditions of eluation suitable for the next immunoglobulin and so forth.

## Claims

1. A process for the separation by affinity chromatography of anti-whooping cough immunoglobulin from a solution of the Cohn's fraction II, obtained from standard plasma, using an agarose resin derivatized with whooping cough anatoxin **characterized in that**:
- the antigen/resin ratio is of 0.8 - 3 expressed as mg of antigen per ml of resin
- the solution containing the immunoglobulin is loaded at a rate of 0.8 cm/h and the column is washed with 15 beds of the buffer consisting of 0.01 M phosphate buffer, 0.15 M NaCl, pH 7.4;
- the elution is performed at a flow rate of 6.7 cm/h with the buffer consisting of 0.3 M glycin HCl q.s., pH2.5;
- the solution is collected and properly stabilized at the registered elution peak.

2. A process for the separation of anti-diphtheria immunoglobulin from a solution of the Cohn's fraction II obtained from standard plasma using a resin derivatized with diphteric anatoxin **characterized in that**:
- the antigen/resin ratio is of 3 - 5.5 expressed as mg of antigen per ml of resin.
- the solution containing the immunoglobulin is loaded at a rate of 0.117 cm/h and the column is washed with 14 beds of the buffer consisting of 0.01 M phosphate buffer, 0.15 M NaCl, pH 7.4;
- the elution is performed at a flow rate of 7.5 cm/h with the buffer consisting of 0.3 M glycin HCl q.s., pH2.5;
- the solution is collected and properly stabilized at the registered elution peak.

## Patentansprüche

1. Verfahren zur Trennung durch Affinitätschromatographie von Anti-Keuchhusten-Immunglobulin aus einer Lösung der Cohn-Fraktion II (erhalten aus Standardplasma) unter Verwendung eines Agaroseharzes, das mit einem Keuchhustenanatoxin derivatisiert wurde, **dadurch gekennzeichnet, dass**:
- das Antigen/Harz-Verhältnis 0,8 - 3 beträgt, ausgedrückt als mg Antigen pro ml Harz
- die das Immunglobulin enthaltende Lösung mit einer Geschwindigkeit von 0,8 cm/h geladen wird und die Säule mit 15 Pufferlagen, bestehend aus 0,01 M Phosphatpuffer, 0,15 M NaCl, pH 7,4 gewaschen wird;
- die Elution mit einer Durchflussgeschwindigkeit von 6,7 cm/h mit einem Puffer bestehend aus 0,3 M Glycin HCl q.s., pH 2,5 durchgeführt wird;
- die Lösung am registrierten Elutionspeak gesammelt und ordnungsgemäß stabilisiert wird.

2. Verfahren zur Trennung von Anti-Diphtherie-Immunglobulin aus einer Lösung der Cohn-Fraktion II, die aus Standardplasma unter Verwendung eines Harzes erhalten wurde, das mit Diphtherie-Anatoxin derivatisiert wurde, **dadurch gekennzeichnet, dass**:
- das Antigen/Harz-Verhältnis 3 - 5,5 beträgt, ausgedrückt als mg Antigen pro ml Harz
- die das Immunglobulin enthaltende Lösung mit einer Geschwindigkeit von 0,117 cm/h geladen wird und die Säule mit 14 Pufferlagen, bestehend aus 0,01 M Phosphatpuffer, 0,15 M NaCl, pH 7,4 gewaschen wird;
- die Elution mit einer Durchflussgeschwindigkeit von 7,5 cm/h mit einem Puffer bestehend aus 0,3 M Glycin HCl q.s., pH 2,5 durchgeführt wird;
- die Lösung am registrierten Elutionspeak gesammelt und ordnungsgemäß stabilisiert wird.

## Revendications

1. Processus de séparation par chromatographie d'affinité d'une immunoglobuline anti-coqueluche depuis une solution de fraction II de Cohn, obtenue à partir de plasma standard, en utilisant une résine d'agarose dérivée avec l'anatoxine de la coqueluche, **caractérisé en ce que**:
- le ratio antigène / résine est de 0,8 - 3 exprimé en milligramme d'antigène par millilitre de résine;
- la solution contenant l'immunoglobuline est chargée à une vitesse de 0,8 cm/h et la colonne est lavée avec 15 lits du tampon consistant en 0,01 M de tampon phosphate, 0,15 M de NaCl, pH 7,4;
- l'élution est réalisée à un débit de 6,7 cm/h avec le tampon consistant en 0,3 M de glycine HCl q.s., pH 2,5;
- la solution est collectée et stabilisée correctement au pic d'élution enregistré.

2. Processus de séparation d'immunoglobuline antidiphtérique depuis une solution de la fraction II de Cohn obtenue à partir de plasma standard en utilisant une résine dérivée avec l'anatoxine diphtérique, **caractérisé en ce que**:
- le ratio antigène / résine est de 3 - 5,5 exprimé en milligramme d'antigène par millilitre de résine;
- la solution contenant l'immunoglobuline est chargée à une vitesse de 0,117 cm/h et la colonne est lavée avec 14 lits du tampon consistant en 0,01 M de tampon phosphate, 0,15 M de NaCl, pH 7,4;
- l'élution est réalisée à un débit de 7,5 cm/h avec le tampon consistant en 0,3 M de glycine HCl q.s., pH 2,5;
- la solution est collectée et stabilisée correctement au pic d'élution enregistré.
